(19) Europäisches Patentamt
European Patent Office
Office européen des brevets

(11) Publication number: **0 001 825 B1**

(12) # EUROPEAN PATENT SPECIFICATION

(45) Date of publication of patent specification: **13.01.82**

(51) Int. Cl.³: **C 07 C 87/60, C 07 C 85/11**

(21) Application number: **78101266.1**

(22) Date of filing: **31.10.78**

(54) **Method of preparing 2,4-difluoroaniline.**

(30) Priority: **31.10.77 US 847187**
**31.10.77 US 847186**

(43) Date of publication of application:
**16.05.79 Bulletin 79/10**

(45) Publication of the grant of the European patent:
**13.01.82 Bulletin 82/2**

(84) Designated Contracting States:
**BE CH DE FR GB LU NL SE**

(56) References cited:

**CH - A - 535 728**
**DE - A - 1 468 395**
**GB - A - 1 360 327**
**GB - A - 1 433 517**
**US - A - 3 900 519**

**Zhur. Obshchei Khim 31, 1229-32 (1961)**

(73) Proprietor: **MERCK & CO. INC.**
**126, East Lincoln Avenue P.O. Box 2000**
**Rahway, New Jersey 07065 (US)**

(72) Inventor: **Tull, Roger James**
**48 Spring Street**
**Metuchen New Jersey 08840 (US)**
Inventor: **Shinkai, Ichiro**
**121 North Cottage Place**
**Westfield New Jersey 07090 (US)**
Inventor: **Weinstock, Leonard Maurice**
**P.O. Box 218**
**Bellemead New Jersey 08504 (US)**

(74) Representative: **Abitz, Walter, Dr.-Ing. et al,**
**Abitz, Morf, Gritschneder P.O. Box 86 01 09**
**D-8000 München 86 (DE)**

**EP 0 001 825 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Method of preparing 2,4-difluoroaniline

1 Field of the Invention

A method of preparing 2,4-difluoroaniline.

2 Description of the Prior Art

G.C. Finger and C.W. Kruse, in *JACS*, 78, 6034 (1956), have described fluorination of 2,3,4-triochloro nitrobenzene with potassium fluoride in dimethylsulfoxide, to form 1,3-difluoro-2-chloro-6-nitrobenzene, but in low yield. Finger et al., in *JACS*, 81, 94 (1959) have described fluorination of 3,4,5-trichloronitro-benzene with potassium fluoride in dimethylformamide, to form 3,5-dichloro-4-fluoronitrobenzene in fairly high yield. However, the art does not suggest the specific fluorination by chlorine replacement to form 2,4-difluoro-5-chloro-nitrobenzene achieved with the method of the present invention, nor the high yield efficiency achieved with phase transfer catalysis in said method.

U.S. Patent No. 3,992,432 described a process for catalyzing heterogeneous ionic organic reactions in a system of multiple liquid phases in which the reactants are located in different phases. The catalysts are certain organic quaternary salts. However, the method of the patent is one involving only liquid phases, especially an organic phase and an aqueous phase, and there is no suggestion of employing a solid phase and a liquid phase as in the method of the present invention.

Christensen et al., in *Chem. Res.*, 74, 351 (1974), and Gokel and Durst, in *Aldrichimica acta*, 9, 3 (1976), describe the use of crown ethers to catalyze a variety of cynthetic reactions by complexing with an insoluble reagent, rendering the entire complex soluble in organic solvents. However, the crown ethers suffer from the disadvantages of limited solubility, cation specificity, and high cost; none of which characterize the phase transfer catalysts of the present invention.

Hydrogenolysis of various halobenzenes with hydrogen over palladium-carbon by M. Kraus and V. Bazant, reported in *Catal., Proc. Int. Congr., 5th 1972*, 2, 1073-84, determined that reactivity decrease occured in the order Br, Cl, F. And R.E. Florin et al., in *J. Res. Natl, Bur. St.*, 62, 119 (1959) found that hydrogenation with hydrogen over palladium-carbon of 2,3,4,5-tetrafluoro-chlorobenzene formed 1,2,3,4-tetrafluorobenzene in fairly high yield. However, when catalytic reduction over palladium black of aromatic fluoro chloro nitro compounds was carried out by N. Vorozhtsov et al., reported in *Zhur. Obshchei Khim.*, 31, 1222-6 (1961), it was found that 2,4-difluoro-5-chloroaniline was formed. Thus, the art does not suggest the catalytic reduction of the method of the present invention whereby the nitro group is reduced to amine and the chlorine substituent is displaced without affecting the fluorine substituents, in a very specific manner.

A commonly employed method in the art currently for preparing 2,4-difluoroaniline is one which prepares 1,3-dichlorobenzene as an intermediate and uses benzene as a starting material. However, this process requires use of $AlCl_3$ catalyst, an expensive material, as well as fractional distillation. The intermediate is then nitrated, and fluorine replacement and reduction produces 2,4-difluoroaniline. The reactions of this conventional method may be illustrated as follows:

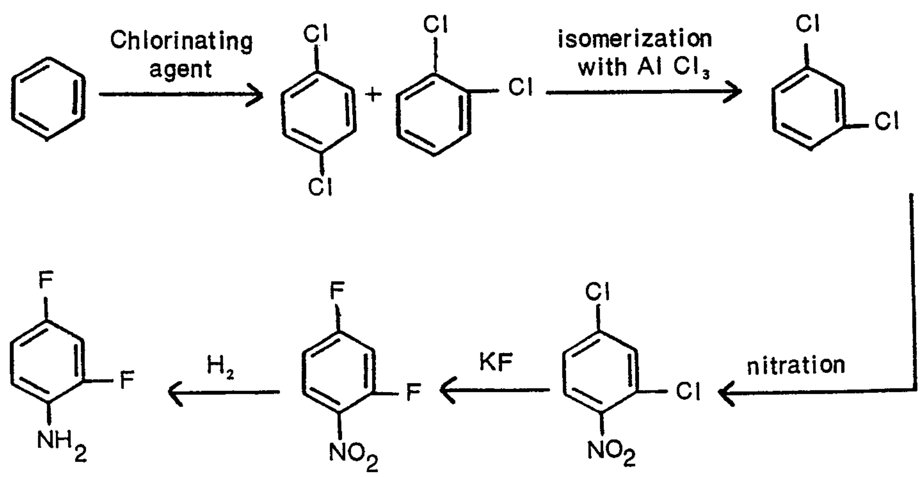


The method of the present invention avoids the expensive isomerization step of the above preocess by employing trichlorobenzene, an inexpensive starting material, with specific chlorine removal at the time of fluorine replacement. GB-A 1,360,327 describes the reaction of 2,4,5-trichloronitrobenzene with a fluorinating agent to form 2,4-difluoro-5-chloronitrobenzene. DE-A 1,468,395 relates to as complete as possible fluorination of aryl perhalides. Therefore, it was not possible to take any sort of pointer to the present selective preparation of 2,4-difluoroaniline from a combination of GB-A 1,360,327 and DE-A 1,468,395.

SUMMARY OF THE INVENTION

The method of the present invention is concerned with preparation of 2,4-difluoroaniline.

The present invention is particularly concerned with a fluorination replacement reaction which employs a solid-liquid phase transfer catalyst to achieve high yields.

More particularly, the method of the present invention comprises two steps characterized by high specificity whereby preparation of 2,4-difluoroaniline is greatly simplified and improved. The first step involves fluorination of 2,4,5-trichloronitrobenzene whereby two of the chlorine substituents are replaced by fluorine in a specific manner to form 2,4-difluoro-5-chloronitrobenzene. The first step is carried out with greatly improved efficiency and high yields through the use of a quaternary compound solid-liquid phase transfer catalyst in the presence of a solid-liquid phase interface. The second step involves hydrogenation of the 2,4-difluoro-5-chloronitrobenzene where the nitro substituent is reduced to amine the chlorine substituent is displaced to form HCl, and the two fluorine substituents remain unaffected. The hydrogenation step is, thus, quite specific.

The method of the present invention, comprising basically two steps, but three separate reactions may be schematically represented as follows:

Step 1. Fluorination of 2,4,5-trichloronitrobenzene

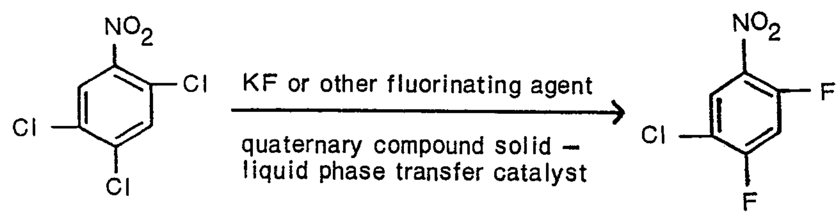


Step 2. Hydrogenation of 2,4-difluoro-5-chloronitrobenzene

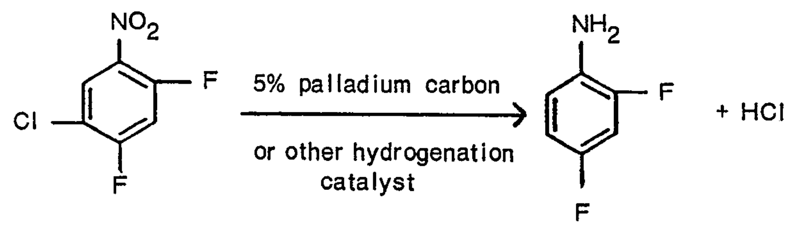


The starting material for the method of the present invention may be 1,2,4-trichlorobenzene, an inexpensive material. This material must then be nitrated to prepare 2,4,5-trichloronitrobenzene. However, it is preferred to employ 2,4,5-trichloronitrobenzene as a starting material since it is fairly readily available and relatively inexpensive.

The fluorination step of the method of the present invention, in addition to employing a quaternary compound solid-liquid phase transfer catalyst, is carried out under essentially anhydrous conditions using a fluorinating agent selected from NaF, KF, CsF, and $C_{1-4}$ alkyl quaternary ammonium fluoride, and mixtures thereof. KF is preferred as the fluorinating agent. Since the presence of water slows the fluorination considerably, the reaction conditions must be substantially anhydrous, although trace amounts of water can be tolerated. Anhydrous conditions are usually maintained by employing an organic solvent reaction medium. Basically any organic solvent medium which will dissolve the 2,4,5-trichloronitrobenzene and in which the fluorinating agent is essentially insoluble, may be deployed. Hydrocarbon solvents are preferred, expecially aromatic hydrocarbons, unsubstituted and substituted with groups independently selected from lower alkyl and halogen, for example, benzene, toluene, xylene, chlorobenzene, *o*-dichlorobenzene and trifluorobenzene. The solvent reaction medium may also comprise a dipolar and aprotic solvent. A number of such solvents are suitable for example, one selected from dimethylsulfoxide, tetramethylsulfone, dimethylformamide, dimethylacetamide, tetramethylurea, dimethylsulfone, and hexamethylphosphoramide. A molar excess of the fluorinating agent, e.g., potassium fluoride, is used, usually of at least five fold in amount. The fluorination reaction is carried out at elevated temperatures of from about 75° to about 225°C., preferably of from about 100° to about 175°C., and for a period of time ranging from about 3 to about 30 hours, usually from 8 to about 20 hours.

As already mentioned above, one unexpected improvement of the present invention comprises use of a quaternary compound solid-liquid phase transfer catalyst during the fluorination step. Accordingly, the reactants for the fluorination step are carried in two separate phases. The solid fluorinating agent, for example KF, is one phase, a solid phase, and the other phase is a liquid phase and

comprises the reactant 2,4,5-trichloronitrobenzene dissolved in an organic solvent in which the solid fluorinating agent is insoluble or only silightly soluble, for example toluene. Two discrete phases thus exist in which the reactants of the fluorination step are separately carried. It is also possible to employ as the liquid phase, the 2,4,5-trichloronitrobenzene reactant itself, in a molten state. The low melting point of this normally solid material makes such a procedure fairly easy to accomplish and, further, dispenses with the need for an organic solvent medium. In this case, the two phases, solid and liquid, would consist of the two reactants themselves, that is, 2,4,5-trichloronitrobenzene and KF, for example.

The solid-liquid phase transfer catalysis occurs essentially by a mechanism of transfer across the phase interface in which the ionic functional group of the fluorinating agent, that is, the $F^{\ominus}$ ion, is transferred from the solid phase to the liquid phase containing the reactant 2,4,5-trichloronitrobenzene. The quaternary compound solid-liquid phase transfer catalyst acts as an acceptor for the ionic functional group from the solid phase, and as a donor of that ionic functional group to the liquid phase. Thus, the quaternary compound acts as a transporting medium or element for the ionic functional group, the F ion.

The quaternary compounds employed as solid liquid phase transfer catalysts in the fluorination step of the method of the present invention may be represented by the formula

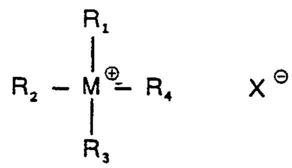

where M is selected from nitrogen and phosphorus; $X^{\ominus}$ is any anion capable of dissociating from the cation $M^{\oplus}$, and is preferably halide; and $R_1$, $R_2$, $R_3$ and $R_4$ are monovalent hydrocarbon radicals selected from alkyl, alkenyl, alkynyl, aryl, alkaryl, aralkyl, and cycloalkyl.

The quaternary compound must be soluble in the liquid phase, that is, the organic solvent medium in which the 2,4,5-trichloronitrobenzene is dissolveed or the 2,4,5-trichloronitrobenzene itself. For example, the quaternary compound must be soluble in toluene, the preferred solvent medium for the liquid phase. Accordingly, $R_1$, $R_2$, $R_3$ and $R_4$ must be selected so that the total number of carbon atoms involved is at least 18, and preferably higher. While some maximum number of total carbon atoms necessarily exists, that number is not generally significant, and, as a practical matter, the maximum number of carbon atoms involved should not be greater than about 60.

While it is preferred to employ a single quaternary compound as the solid-liquid phase transfer catalyst, it is possible to employ mixtures of the quaternary compounds in this way.

Examples of quaternary compounds which may be employed as transfer catalysts in the fluorination step of the method of the present invention are the following:

trioctylethlammonium bromide
tridecylmethylammonium chloride
didodecyldimethylammonium chloride
tetraheptylammonium iodide
dioctadecyldimethylámmonium chloride
tridecylbenzylammonium chloride
triphenyldecylphosphonium iodide
tributylhexadecylphosphonium iodide

It is also possible to employ quaternary compounds in which one or more of $R_1$, $R_2$, $R_3$ and $R_4$ are mixtures of long chain alkyl groups within a specified range. Thus, in a preferred quaternary compound, Aliquat 336 (Trade Mark) available from McKerson Corp., Minneapolis, Minnesota 55408, one R group is methyl while the remaining R groups are various long chain alkyl groups in the range of eitht to twelve carbon atoms, and the compound may be represented by the formula: $(n\text{-}C_8\text{-}C_{12})_3NCH_3^{\oplus}Cl^{\ominus}$. The compound has an average molecular weight of about 500.

All of the above quaternary compounds are genreally satisfactory for use in the method of the present invention, and will usually be, as they must, inert or non-reactive with respect to the various materials employed in the reaction mixture, except the reactants themselves.

The amount of quaternary compound which it is necessary to employ will vary, depending, among other things, upon the solvent medium which is selected and the desired rate of reaction. Generally, however, the amount employed will usually be from about 10 to about 75 weight percent, based on weight of 2,4,5-trichloronitrobenzene in the solvent medium of the liquid phase.

The 2,4-difluoro-5-chloronitrobenzene formed by the fluorination step described above is next subjected to the catalytic hydrogenation step of the method of the present invention. The hydrogenation catalyst employed is preferably a 5% palladium carbon catalyst. This catalyst is an activated carbon substrate impregnated with 5% by weight of palladium. The manner of impregnation is not

critical and palladium carbon catalysts prepared by various conventional processes are suitable. A 5% palladium carbon catalyst available from Engelhard Industries, Inc. has been found especially suitable. A 10% palladium carbon catalyst has been found to give results essentially similar to those obtained with the 5% palladium catalyst. Other hydrogenation catalysts which may be employed are $Ni/Cr_2O_3$ or Raney nickel catalysts, preferably on an activated carbon substrate. The amount of catalyst employed will usually be from about 5% to about 25% by weight of the amount 2,4-difluoro-5-chloronitrobenzene starting material. The catalyst material and starting material are added to a suitable solvent, for example, methanol or higher alkanol, or aqueous mixtures of these together with sodium acetate or other mild base to neutralize the HCl formed during the reaction. Hydrogen is them bubbled through the reaction solution by means of suitable apparatus, for example, a Parr hydrogenater. The reaction solution is stirred or shaken while hydrogen is passed through the solution.

The reaction solution is initally maintained at a temperature of from about 0° to about 100°C. However, reduction of the nitro group is exothermic and cooling may be required if the apparatus employed does not permit dissipation of the heat generated. Otherwise, by starting at a sufficiently low temperature, it is possible to run the total hydrogenation process straight through without addition or extraction of any heat. The chlorine removal portion of the hydrogenation process requires temperatures between about 20° to 100°C. Thus, it is possible, but not necessary, to carry out the hydrogenation process in essentially two stages: nitro group reduction followed by chlorine removal.

The hydrogen pressure during hydrogenation is usually maintained between about 294 and 981 kPa most usually about 294 kPa. Stoichiometrially, four moles of hydrogen are required for hydrogenation of 2,4-difluoro-5-chloronitrobenzene. As a practical matter, hydrogen is passed through the reaction solution until the reaction is complete, since all the hydrogen which is required for the hydrogenation reaction will be consumed, and any excess or additional amounts will have no adverse consequences. Thus, the time required for the hydrogenation step will depend on a number of factors, including hydrogen pressure and temperature of the reaction solution. Usually, hydrogenation will be complete in from about 1 to about 5 hours.

Typically, the hydrogenation may be carried out as follows: after at least about three moles of hydrogen have been passed through the solution, the solution is heated to a temperature of from 50° to 70°C. and an additional at least two moles of hydrogen are passed through the solution.

## EXAMPLE 1

Step 1: Preparation of 2,4-difluoro-5-chloronitrobenzene:
Fluorination of 2,4,5-trichloronitrobenzene

5g. (2,208 x $10^{-2}$ mole) of 2,4,5-trichloronitrobenzene was added to 30ml. of tetramethyl sulfone together with 6.4g. (1.102 x $10^{-1}$ mole) of anhydrous potassium fluoride. The reaction mixture was then heated at 170—180°C. for 19 hours. The reaction mixture was them subjected to steam distillation and the distillate of about 150ml. was extracted with ether five times at 20ml. per extraction. The extract was dried over $Na_2SO_4$ to give 1,56g. of a yellow oil. The residue after extraction was treated with 150ml. water and then extracted with ether five times at 40ml. per extraction. The extract was dried over $Na_2SO_4$ to give a yellow oil. The yellow oil was purified on a column of silica gel (3 x 25cm.) by first eluting with 5% $CHCl_3$/n-hexane (1:9) to yield 310mg. of 2,4,5-trichloronitrobenzene starting material, and then 10% $CHCl_3$/n-hexane (1:9) to yield 670mg. of 3,4-difluoro-5-chloronitrobenzene. Both results were confirmed by NMR and TLC. The total yield of 2,4-difluoro-5-chloronitrobenzene was 2.23g. or 52%.

Step 2: Preparation of 2,4-difluoroaniline:
Hydrogenation of 2,4-difluoro-5-chloronitrobenzene

1.0g. (5.17 x $10^{-3}$ mole) of 2,4-difluoro-5-chloronitrobenzene, together with 450mg. (5.49 x $10^{-3}$ mole) of sodium acetate, was added to 30ml. of methanol. 100mg. of 5% palladium-carbon catalyst was added and hydrogen was then passed through the reaction mixture for a period of 3 hours in a total amount of 20 x $10^{-3}$ moles. The reaction mixture was then heated to 60°C. and hydrogen was allowed to pass through the mixture for the next 20 hours, in a total amount of 5.2Kg. The reaction mixture was then filtered to remove the catalyst material and sodium acetate. The final product was separated by evaporation of the solvent under vacuum, followed by water washing of the product. The product was then introduced into an insoluble solvent, followed by distillation. The yield was approximately 70%.

## EXAMPLE 2

Preparation of 2,4-difluoroaniline:
Hydrogenation of 2,4-difluoro-5-chloronitrobenzene

2.0g. (1.03 x $10^{-2}$ mole) of 2,4-difluoro-5-chloronitrobenzene together with 900mg. (1.1 x $10^{-2}$ mole) of sodium acetate, and 0.2g. of 5% palladium-carbon catalyst, was added to 30ml. of methanol. 18.1Kg of hydrogen was passed through the reaction mixture for 3 hours, after which it was heated to 60°C. During the following 6 hours and 14 minutes an additional 5.4Kg of hydrogen was passed through the reaction mixture. The catalyst and sodium acetate were removed by filtration and washed with 10ml. of methanol. The reaction mixture was then concentrated to about 5ml. and poured into

40ml, of a 5% $Na_2CO_3$ solution. The solution was extracted with chloroform three times at 20ml. per extract. The extract was dried over $Na_2SO_4$. The solvent was evaporated under reduced pressure and the residue was purified by vacuum distillation. The yield of final product was 640mg., which was 48%.

EXAMPLE 3

Preparation of 2,4-difluoro-5-chloronitrobenzene:
Fluorination of 2,4,5-trichloronitrobenzene

5.0g. (2.208 x $10^{-2}$ mole) of 2,4,5-trichloronitrobenzene, 2.8g (4.82 x $10^{-2}$ mole) of anhydrous potassium fluoride, 50ml. of dimethylsulfoxide, and 3.0g. (0.006 mole) of Aliquat 336 (Trade Mark), a tetra-alkyl ammonium chloride, were admixed together and heated at 100°C. for 1 hr., and at 120°C. for 3 hrs. The mixture was then poured into 100ml. of hot water and the reaction product was separated by steam distillation. The 2.6 litres of distillate was then extracted with 80ml. of ether for each of a total of six times. The extract was dried over $Na_2SO_4$ to give 3.01g. of a crude, pale yellow oil, a yield of 70.5%. Results were confirmed by thin layer chromatography and gas chromatography, which indicated the final product to be 78.2% 2,4-difluoro-5-chloronitrobenzene.

EXAMPLE 4

Preparation of 2,4-difluoro-5-chloronitrobenzene:
Fluorination of 2,4,5-trichloronitrobenzene

5.0g. (2.208 x $10^{-2}$ mole) of 2,4,5-trichloronitrobenzene, 2.8g. (4.82 x $10^{-2}$ mole) of anhydrous potassium fluoride, 50ml. of dimethylsulfoxide, and 3.0g (0.006 mole) of Aliquat 336 (Trade Mark), a tetra-alkyl ammonium chloride, were admixed together and heated at 90 — 100°C. for 17 hours. The reaction mixture was then poured into 20ml. of water and the reaction product was separated by steam distillation. Approximately 2 liters of distillate was extracted twice with 200ml. of ether for each extraction. The combined extract was dried to give a crude oil. The yield of final produce was 2.98g. or 69.7%. Results were confirmed by thin layer and gas chromatography for 2,4-difluoro-5-chloronitrobenzene.

EXAMPLE 5

Preparation of 2,4-difluoro-5-chloronitrobenzene:
Fluorination of 2,4,5-trichloronitrobenzene

5.0g. (2.208 x $10^{-2}$ mole) of 2,4,5-trichloro-nitrobenzene, 2.8g. (4.82 x $10^{-2}$ mole) of anhydrous potassium fluoride, 50ml. of toluene, and 3.0g. (0.006 mole) of Aliquat 336 (Trade Mark), a tetra-alkyl ammonium chloride, were admixed together and heated at 90—95°C. for 17 hours. The mixture was then maintained at 100—105°C. for 8 hours, after which it was boiled under reflux for 16 hours. Then, 560mg. of additional anhydrous potassium fluoride was added and the mixture was boiled under reflux for 23 hours. The reaction product was next separated by steam distillation. Two liters of distillate was extracted with ether and the extract dried to give a crude, yellow to orange, oil. The yield of final product was 3.96g. or 92.7%. Gas chromatographic analysis indicated that the final product contained 81.6% of 2,4-difluoro-5-chloronitrobenzene.

EXAMPLE 6

Preparation of 2,4-difluoro-5-chloronitrobenzene:
Fluorination of 2,4,5-trichloronitrobenzene

20g. (8.83 x $10^{-2}$ mole) of 2,4,5-trichloronitrobenzene was dissolved in 25ml. of toluene. Next, 13.0g. (2.24 x $10^{-1}$ mole) of potassium fluoride was first dried overnight at 80°C. under vacuum, and then added to 75ml. of toluene with which it was axetropically distilled for 1 hour. Then, 4.0g. (8 x $10^{-3}$ mole) of Aliquat 336 (Trade Mark), a tetra-alkyl ammonium chloride, was added to 20ml. of toluene and this solution was added to the potassium fluoride and toluene mixture and the total mixture was axeotropically distilled for 1 hour. The toluene solution of 2,4,5-trichloronitrobenzene was then slowly added to this total mixture. The reaction mixture was then refluxed for 119 hours, after which 15g. of dimethylsulfoxide was added and the reaction mixture refluxed for 9 hours. The reaction mixture was then poured into 150ml. of saturated NaCl solution, and the reaction product was separated by steam distillation to give a crude, orange oil. Yield of final product was 15.1g. or 88.4%. The final produce was then distilled under vacuum to give a pale yellow liquid having a boiling point of 70—72°C. The yield of this final product was 14.8g. or 86.6%.

EXAMPLE 7

Preparation of 2,4-difluoro-5-chloronitroaniline:
Fluorination of 2,4,5-trichloronitrobenzene

A mixture of 20g. (8.83 x $10^{-2}$ mole) of 2,4,5-trichloronitrobenzene, 13g. (2.24 x $10^{-1}$ mole of anhydrous potassium fluoride, and Aliquat 336 (Trade Mark), (4.0g., 8 x $10^{-3}$ mole) in 75ml. of dimethylsulfoxide was heated at 95—100°C. for 23 hours. The reaction produce was next separated by steam distillation yielding 2 liters of distillate. The distillate was extracted with dichloromethane four times using 70ml. for each extraction; washed with water, then 5% sodium hydroxide, and again water; and then dried over sodium sulfate. Evaporation of the solvent gave 16.85g. of crude 2,4-difluoro-5-chloronitrobenzene, a yield of 98.6%. Gas chromatography indicated a purity of 91%.

EXAMPLE 8

Preparation of 2,4-difluoroaniline:
Hydrogenation of 2,4-difluoro-5-chloronitrobenzene

A solution of 2,6-difluoro-5-chloronitrobenzene (2.0g., 1.03 x $10^{-2}$ mole) and triethylamine (2.1g., 2.06 x $10^{-2}$ mole) was hydrogenated over 10% palladium carbon catalyst for 18 minutes at room temperature and for an additional 4 hours at 60°C. The catalyst was removed by filtration and washed twice with methanol, using 16ml. for each wash. The solvent was evaporated and the residue was treated with dichloromethane. The resulting mixture was washed with 15ml. of water, 70ml. of ammonium hydroxide solution and then 15ml. of water, and dried over $Na_2SO_4$. Evaporation of the remaining solvent gave 1.25g. of a yellow oil, a yield of 94%. Gas chromatography established the reaction product to contain 89.5% 2,4-difluoro-aniline.

The 2,4-difluoroaniline prepared by the method of the present invention is a well known starting material and intermediate for a number of organic sytheses. See *The Merck Index*, ninth edition, pg. 415 (1976). Expecially, the 2,4-difluoroaniline prepared by the method of the present invention is a useful starting material for preparation of 2′,4′-difluoro-4-hydroxy-[1,1′-biphenyl]-3-carboxylic acid, a valuable anti-inflammatory and analgesic agent for therapeutic use.

## Claims

1. A method preparing 2,4-difluoroaniline comprising the steps of

(a) fluorinating 2,4,5-trichloronitrobenzene in a solid-liquid phase system, under anhydrous conditions, wherein the solid phase comprises a fluorinating agent selected from NaF, KF, CsF, $C_{1-4}$ alkyl quaternary ammonium fluoride, and mixtures thereof; and the liquid phase comprises an organic solvent in which the 2,4,5-trichloronitrobenzene is soluble and in which the fluorinating agent is essentially insoluble; and the fluorination is carried out in the presence of one or more quaternary compound solid-liquid phast transfer catalysts of the formula

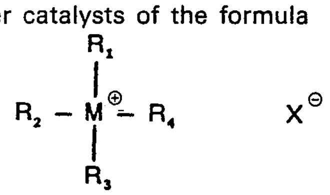

where M is nitrogen or phosphorus; $X^{\ominus}$ is an anion capable of dissociating from the $M^{\oplus}$ cation; and $R_1$, $R_2$, $R_3$ and $R_4$ are monovalent hydrocarbon radicals selected from alkyl, alkenyl, alkynyl, aryl, aralkyl, alkaryl, and cycloalkyl, which together have a total of at least 18 carbon atoms; to form 2,4-difluoro-5-chloronitrobenzene; and

(b) hydrogenating the 2,4-difluoro-5-chloronitrobenzene with hydrogen in the presence of a hydrogenation catalyst to form 2,4-difluoroaniline.

2. The method of Claim 1 wherein the organic solvent is a hydrocarbon solvent.

3. The method of Claim 2 wherein the hydrocarbon solvent is selected from aromatic hydrocarbons, unsubstituted and substituted with groups independently selected from lower alkyl and halogen.

4. The method of Claim 3 wherein the hydrocarbon solvent is toluene.

5. The method of Claim 1 wherein the fluorinating agent is KF.

6. The method of Claim 1 wherein the quaternary compound is $(n\text{-}C_8\text{-}C_{12})_3NCH_3^{\oplus}Cl^{\ominus}$.

7. The method of Claim 1 wherein the hydrogenation catalyst is palladium impregnated activated carbon.

## Revendications

1. Un procédé de préparation de difluoro-2,4-aniline, comprenant les étapes selon lesquelles

(a) on fluore du trichloro-2,4,5-nitrobenzène dans un système de phases solide-liquide, dans des conditions anhydres, où la phase solide comprend un agent de fluoration choisi parmi NaF, KF, CsF, un fluorure de $C_{1-4}$alcoyl ammonium quaternaire et leurs mélanges; et la phase liquide comprend un solvant organique dans lequel le trichloro-2,4,5-nitrobenzène est soluble et dans lequel l'agent de fluoration est sensiblement insoluble; et la fluoration est conduite en présence d'un ou plusieurs composés quaternaires servant de catalyseur par transfert de phase solide-liquide, de la formule

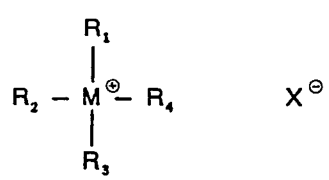

dans laquelle M est de l'azote ou du phosphore; $X^{\ominus}$ est un anion capable de se dissocier du cation $M^{\oplus}$; et $R_1$, $R_2$, $R_3$ et $R_4$ sont des radicaux d'hydrocarbures monovalents choisis parmi les radicaux alcoyle, aclényle, alcynyle, aryle, aralcolyle, alcaryle et cycloalcoyle qui ont ensemble un total d'au moins 18 atomes de carbone; pour former du difluoro-2,4-chloro-5-nitrobenzène; et

(b) on hydrogène le difluoro-2,4-chloro-5-nitrobenzène avec de l'hydrogène en présence d'un catalyseur d'hydrogénation de manière à former de la difluoro-2,4-aniline.

2. Un procédé selon la revendication 1, caractérisé en ce que le solvant organique est un solvant hydrocarboné.

3. Un procédé selon la revendication 2, caractérisé en ce que le solvant hydrocarboné est choisi parmi des hydrocarbures aromatiques, non-substitués ou substitués par des groupes choisis indépendamment parmi les radicaux alcoyle inférieur et les halogènes.

4. Un procédé selon la revendication 3, caractérisé en ce que le solvant hydrocarboné est du toluène.

5. Un procédé selon la revendication 1, caractérisé en ce que l'agent de fluoration est KF.

6. Un procédé selon la revendication 1, caractérisé en ce que le composé quaternaire est $(n\text{-}C_8\text{-}C_{12})_3NCH_3^{\oplus}Cl^{\ominus}$.

7. Un composé selon la revendication 1, caractérisé en ce que le catalyseur d'hydrogénation est du charbon activé imprégné de palladium.

## Patentansprüche

1. Methode zur Herstellung von 2,4-Difluoranilin durch folgende Stufen

(a) Fluorierung von 2,4,5-Trichlornitrobenzol in einem Fest-Flüssig-Phasensystem unter wasserfreien Bedingungen, in dem die feste Phase ein Fluorierungsmittel enthält, ausgewählt aus NaF, KF, CsF, $C_{1-4}$-Alkyl-quaternärem Ammoniumfluorid, und Gemischen davon; und die flüssige Phase ein organisches Lösungsmittel enthält, in dem das 2,4,5-Trichloronitrobenzol löslich ist und in dem das Fluorierungsmittel im wesentlichen unlöslich ist; und Durchführen der Fluorierung in Anwesenheit von einer oder mehreren quaternären Fest-Flüssig-Phasen-Transfer-Katalysatoren-Verbindung mit der Formel

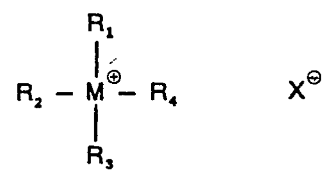

worin M stickstoff oder Phosphor ist; $X^{\ominus}$ ein Anion ist, geeignet zur Dissoziation von dem $M^{\oplus}$-Kation; und $R_1$, $R_2$, $R_3$ und $R_4$ einwertige Kohlenwasserstoffreste sind, ausgewählt aus Alkyl, Alkenyl, Alkinyl, Aryl, Aralkyl, Alkaryl und Cycloalkyl, die zusammen insgesamt mindestens 18 Kohlenstoffatome haben; unter Bildung von 2,4-Difluor-5-chlornitrobenzol; und

(b) Hydrieren des 2,4-Difluor-5-chlornitrobenzols mit Wasserstoff in Anwesenheit eines Hydrierungskatalysators unter Bildung von 2,4-Difluoranilin.

2. Methode nach Anspruch 1, bei der das organische Lösungsmittel ein Kohlenwasserstofflösungsmittel ist.

3. Methode nach Anspruch 2, bei der das Kohlenwasserstofflösungsmittel ausgewählt wird aus aromatischen Kohlenwasserstoffen, unsubstituiert und substituiert mit Gruppen, die unabhängig ausgewählt werden aus Niedrigalkyl und Halogen.

4. Methode nach Anspruch 3, bei der das Kohlenwasserstofflösungsmittel Toluol ist.

5. Methode nach Anspruch 1, bei der das Fluorierungsmittel KF ist.

6. Methode nach Anspruch 1, bei der die quaternäre Verbindung $(n\text{-}C_8\text{-}C_{12})_3\ NCH_3^{\oplus}Cl^{\ominus}$ ist.

7. Methode nach Anspruch 1, bei der der Hydrierungskatalysator mit Palladium imprägnierte Aktivkohle ist.